# EUROPEAN PATENT SPECIFICATION

(11) **EP 1 880 727 B1**
(45) Date of publication and mention of the grant of the patent: **14.09.2011**
(21) Application number: 07013448.1
(22) Date of filing: 09.07.2007
(51) Int. Cl.: A61K 36/185, A61K 35/74, A61K 31/19, A61K 9/00

(54) **Topical vaginal pharmaceutical compositions**
Topische pharmazeutische Zusammensetzungen für die Vagina
Compositions pharmaceutiques vaginales topiques

(30) Priority: 20.07.2006 IT MI20061421
(43) Date of publication of application: 23.01.2008
(73) Proprietor: Velleja Research SRL, 29010 Pontenure (PC) (IT)
(72) Inventor: Di Pierro, Francesco, 20090 Trezzano Sul Naviglio (Milano) (IT)
(74) Representative: Minoja, Fabrizio

(56) References cited:
- WO-A2-2004/035072
- GB-A- 2 112 285
- PIERRO F D I ET AL: "Protective effects of Krameria triandra Ruiz in vaginal diseases with Candida" GIORNALE ITALIANO DI OSTETRICIA E GINECOLOGIA 200804 IT, vol. 30, no. 4, April 2008 (2008-04), pages 115-116, XP009129241 ISSN: 0391-9013

## Description

The present invention relates to topical vaginal pharmaceutical formulations with a local action, comprising lactobacilli, lactic acid and rhatany extracts.

### PRIOR ART

Under normal conditions, the vaginal mucosa is inhabited by a rich, varied flora of micro-organisms, which are very often tissue-specific. This microbial flora can be conveniently divided into two categories: "resident" flora, which is almost invariably present and, if altered, can be restored; and "transient" flora, which can colonise the host for short periods, but tends to be eliminated by competition from the resident micro-organisms or the host' s defence mechanisms. Transient flora can also consist of potentially pathogenic micro-organisms, but this is not very common in healthy individuals.

Normal vaginal flora represents an effective barrier that reduces the probability of colonisation of the host surfaces by pathogenic micro-organisms (a phenomenon known as "colonisation-resistance").

In adults, antibiotic treatment, stress, dietary imbalance and disease alter the quality and quantity of the beneficial microbial vaginal flora, thus exposing the vaginal mucosa to potential infections. The problem of achieving a fast, efficient re-colonisation process consequently arises. This process is significantly facilitated by "probiotics". These products, normally used as nutritional supplements in the prophylaxis of gastro-enteric disorders, can also be used "topically", for instance applied directly to the mucosa, to prevent vaginal infections.

Probiotics are live micro-organisms which favourably influence the health of the host by improving the microbiological balance. Probiotic organisms must be:
- normal constituents of human flora or highly adaptable to that habitat;
- capable of specific adherence to the vaginal epithelium;
- easy to use in clinical practice.

Probiotic bacteria which meet that definition are mainly represented by lactic-acid-producing bacteria, and in particular lactobacilli (acidophilic). These live microbial agents are capable of rapid colonisation, which soon leads to performance of their functions:
1) protection by means of direct antagonism towards potentially pathogenic populations (inhibition of adherence to the epithelium; production of bacteriocines; competition for nutrients and substrates: creation of unfavourable pH conditions and redox microenvironments);
2) stimulation and education of the immune system (macrophagic activation, boosting of natural killer cells, increased production of interferons, and balancing of T-helper 1 and 2 populations).

The normal vaginal secretion has an acid pH of around 4.5; this acidity is due to the production of lactic acid from glycogen resulting from the action of Doderlein's bacillus (a particular lactobacillus). In order for a sufficient quantity of glycogen to be present in the vaginal epithelium, a normal level of estrogens is required; consequently, at times of life when oestrogen production is low (pre-pubescent or post-menopausal age), the vaginal pH is less acid, which makes colonisation by pathogenic germs easier. As local treatment based on estrogens is not very practical, this problem is tackled with "topical" use of lactic acid which, by reducing the pH to values of around 4, in itself constitutes a considerable physical barrier to colonisation by pathogens.

Rhatany (*Krameria triandra*) is a herbaceous plant belonging to the Rosaceae family, originating in the Bolivian and Peruvian Andes. It is used in traditional medicine for its astringent, antibacterial and antifungal action, in the treatment of inflammation of the gums and oral mucosa, dermatitis, haemorrhoids and anal fistulas, and as an anti-diarrhoeal agent.

The active part of the plant, consisting of the dried roots, bark and leaves, mainly contains neolignans with an antimicrobial action against Gram-negative and Gram-positive bacteria and anaerobic strains, and an antifungal action (specifically, eupomatenoid); other polyphenols, flavonoids and tannins, in particular Krameriatannin (or rhatany-tannic acid), benzofuran derivatives (rhatany-phenol I and II), lignans and neolignans with antibacterial activity, nor-lignans with an antifungal action, and rhatanin, a methylated derivative of tyrosine. Rhatany is mainly used in the form of the dried extract obtained by extraction, typically with chlorinated solvents, esters and aliphatic and aromatic ketones.

### DESCRIPTION OF THE INVENTION

The present invention relates to topical vaginal formulations, preferably vaginal tablets comprising lactobacilli, lactic acid and rhatany extracts.

More particularly, the compositions according to the invention contain *Lactobacillus acidophilus,* lactic acid and dried rhatany extract.

The compositions according to the invention provide a strong barrier to the development of common vaginal infections, and allow an ideal commensal microbial flora to be maintained in both women of childbearing age and menopausal women.

The compositions according to the invention will contain *Lactobacillus acidophilus* in an amount from 100,000 to 20 billion per tablet, preferably 1 billion/tablet; lactic acid in an amount from 1 to 50 mg, preferably 15 mg; and rhatany extract in an amount from 1 to 50 mg.

According to this invention, the tablet, inserted deeply into the vagina, disintegrates within 5 minutes and releases the active constituents, restoring and stabilising the vaginal pH in a few minutes to acidity values of around 4-4.5, which are considered ideal to protect against common infections. In the next few minutes, colonisation takes place; the lactobacilli proliferate, aided by the immediate reduction in pH caused by the release of lactic acid, and rebalance the numerical ratios with the remaining portion of the commensal flora if necessary.

The consequences of this stabilisation are evident well-being of the vaginal area and a reduced risk of bacterial, fungal and mycoplasmal infections.

The compositions according to the invention, which are administered vaginally, will be prepared according to conventional methods well known in pharmaceutical technology, such as those described in "Remington's Pharmaceutical Handbook", Mack Publishing Co., N.Y., USA, together with suitable pharmaceutically acceptable excipients.

The following examples illustrate the invention in greater detail.

### EXAMPLE 1 - 900 MG VAGINAL TABLETS

| **INGREDIENT** | **UNIT DOSE** | **%** |
|---|---|---|
| | **Mg** | |
| | | |
| *Lactobacillus acidophilus* 150 billion CFU/g | 66.667 | 7.41 |
| 60% lactic acid | 15.000 | 1.67 |
| Rhatany extract | 18.000 | 2.00 |
| Mannitol | 772.333 | 85.81 |
| Crosslinked sodium carboxymethylcellulose | 20.000 | 2.22 |
| Magnesium stearate | 8.000 | 0.89 |
| **TOTAL** | **900** | |

### EXAMPLE 2 - 900 MG VAGINAL TABLETS

| **INGREDIENT** | **UNIT DOSE** | **%** |
|---|---|---|
| | **Mg** | |
| | | |
| *Lactobacillus acidophilus* 150 billion CFU/g | 66.667 | 7.41 |
| 60% lactic acid | 15.000 | 1.67 |
| Rhatany extract | 18.000 | 2.00 |
| Mannitol | 572.333 | 63.59 |
| Starch | 200.000 | 22.22 |
| Crosslinked sodium carboxymethylcellulose | 20.000 | 2.22 |
| Magnesium stearate | 8.000 | 0.89 |
| **TOTAL** | **900** | |

## Claims

1. Topical vaginal compositions comprising lactobacilli, lactic acid and rhatany extracts.

2. Compositions as claimed in claim 1, containing *Lactobacillus acidophilus*, lactic acid and dried rhatany extract.

3. Compositions as claimed in claims 1 and 2, containing *Lactobacillus acidophilus* in an amount from 100,000 to 20 billion per tablet; lactic acid in an amount from 1 to 50 mg; dried rhatany extract in an amount from 1 to 50 mg.

4. Compositions as claimed in the preceding claims, containing *Lactobacillus acidophilus* in an amount of 1 billion/tablet; lactic acid in an amount of 15 mg; dried rhatany extract in an amount of 18 mg.

5. Compositions as claimed in the preceding claims, in the form of vaginal tablets, vaginal capsules or pessaries.

## Patentansprüche

1. Topische Zusammensetzungen für den vaginalen Bereich, umfassend Lactobazillen, Milchsäure und Ratanhia-Extrakte.

2. Zusammensetzungen gemäß Anspruch 1, enthaltend *Lactobacillus acidophilus*, Milchsäure und getrocknetes Ratanhla-Extrakt.

3. Zusammensetzungen gemäß Anspruch 1 und 2, enthaltend *Lactobacillus acidophilus* in einer Menge von 100 000 bis 20 Milliarden pro Tablette; Milchsäure in einer Menge von 1 bis 50 mg; getrocknetes Ratanhia-Extfakt in einer Menge von 1 bis 50 mg.

4. Zusammensetzungen gemäß den vorangehenden Ansprüchen, enthaltend *Lactobacillus acidophilus* in einer Menge von 1 Milliarde/Tablette; Milchsäure in einer Menge von 15 mg; getrocknetes Ratanhia-Extrakt in einer Menge von 18 mg.

5. Zusammensetzungen gemäß den vorangehenden Ansprüchen in Form von vaginalen Tabletten, vaginalen Kapseln oder Pessaren.

## Revendications

1. Compositions vaginales topiques comprenant des lactobacilles, de l'acide lactique et des extraits de ratanhia.

2. Compositions selon la revendication 1, contenant *Lactobacillus acidophilus*, de l'acide lactique et de l'extrait de ratanhia séché.

3. Compositions selon les revendications 1 et 2, contenant *Lactobacillus acidophilus* en une quantité de 100 000 à 20 milliards par comprimé ; de l'acide lactique en une quantité de 1 à 50 mg ; de l'extrait de ratanhia séché en une quantité de 1 à 50 mg.

4. Compositions selon l'une quelconque des revendications précédentes, contenant *Lactobacillus acidophilus* en une quantité d'un milliard/comprimé ; de l'acide lactique en une quantité de 15 mg ; de l'extrait de ratanhia séché en une quantité de 18 mg.

5. Compositions selon l'une quelconque des revendications précédentes, sous la forme de comprimés vaginaux, de capsules vaginales ou de pessaires.
